Europäisches Patentamt

European Patent Office

Office européen des brevets

(11) Veröffentlichungsnummer: **0 449 016 A1**

## (12) EUROPÄISCHE PATENTANMELDUNG

(21) Anmeldenummer: 91103629.1

(22) Anmeldetag: 09.03.91

(51) Int. Cl.5: **C09C 1/00**, C09D 5/00, A61K 7/00, C08K 9/04, C09B 63/00, C09C 3/08

Die Bezeichnung der Erfindung wurde geändert (Richtlinien für die Prüfung im EPA, A-III, 7.3).

(30) Priorität: 24.03.90 DE 4009567

(43) Veröffentlichungstag der Anmeldung: 02.10.91 Patentblatt 91/40

(84) Benannte Vertragsstaaten:
BE CH DE ES FR GB IT LI NL

(71) Anmelder: MERCK PATENT GESELLSCHAFT MIT BESCHRÄNKTER HAFTUNG
Frankfurter Strasse 250
W-6100 Darmstadt(DE)

(72) Erfinder: Glausch, Ralf, Dr.
Reuterallee 10
W-6100 Darmstadt(DE)

(54) Mit organischen Farbstoffen beschichtete Effektpigmente.

(57) Effektpigmente auf Basis von mit Phthalocyanin- oder Metallphthalocyaninfarbstoffen beschichteten Interferenzpigmenten, dadurch gekennzeichnet, daß die Farbstoffe ohne Zuhilfenahme von Bindemittelsystemen oder anderen Hilfsstoffen als fest anhaftende Schicht auf den Pigmenten aufgebracht sind, eignen sich in hervorragender Weise zur Verwendung in Formulierungen wie Lacken, Farbstoffen, Kunststoffen und Kosmetika.

EP 0 449 016 A1

Die Erfindung betrifft Effektpigmente auf Basis von mit Phthalocyanin- oder Metallphthalocyaninfarbstoffen beschichteten Interferenzpigmenten, wobei die Farbstoffe ohne Hilfsstoffe als fest anhaftende Schicht auf den Pigmenten aufgebracht sind.

Es sind verschiedene Verfahren zur Einfärbung von Pigmenten mit organischen Farbstoffen bekannt, z.B. einfaches Mischen von Pigment und Farbstoff (US 4.755.229), Suspendieren von Pigment in Farbstofflösung (US 3.311.485), Aufpfropfen (EP 01 03 986) und Adhäsion mit Hilfe eines Bindemittelsystems (EP 02 20 617).

Insbesondere ist eine dauerhafte Beschichtung mit Phthalocyaninfarbstoffen bisher ausschließlich unter Zuhilfenahme von Bindemitteln, z.B. Polymeren wie beispielsweise in den Offenlegungsschriften EP 220617, JP 59 47 240 und dem US Patent 4.755.229, oder unter Mitfällung von Metallhydroxiden, wie in dem tschechischen Patent CZ 244,792 beschrieben, gelungen.

Es wurde nun überraschenderweise gefunden, daß ohne Beteiligung von Hilfsstoffen eine dauerhafte Beschichtung von Phthalocyanin- und Metallphthalocyaninfarbstoffen auf Interferenzpigmenten erzielt werden kann, wenn man eine Pigmentsuspension in der Farbstofflösung mit einem Lösungsmittel zusammenbringt, in welchem der Farbstoff im wesentlichen unlöslich ist.

Gegenstand der Erfindung sind daher Effektpigmente auf Basis von mit Phthalocyanin- oder Metallphthalocyaninfarbstoffen beschichteten Interferenzpigmenten, dadurch gekennzeichnet, daß die Farbstoffe ohne Zuhilfenahme von Bindemittelsystemen oder anderen Hilfsstoffen als fest anhaftende Schicht auf den Pigmenten aufgebracht sind.

Gegenstand der Erfindung ist daher auch ein Verfahren zur Herstellung von Efektpigmenten auf Basis von mit Phthalocyanin- oder Metallphthalocyaninfarbstoffen beschichteten Interferenzpigmenten, dadurch gekennzeichnet, daß man eine Suspension des Pigments in einer Lösung des Farbstoffs herstellt und die so erhaltene Suspension mit einem Lösungsmittel zusammenbringt, in welchem der Farbstoff zum überwiegenden Teil unlöslich ist.

Gegenstand der Erfindung ist weiterhin die Verwendung der erfindungsgemäßen Effektpigmente in Formulierungen wie Lacken, Farbstoffen, Kunststoffen und Kosmetika.

Gegenstand der Erfindung sind schließlich Formulierungen, die die erfindungsgemäßen Effektpigmente enthalten.

Die erfindungsgemäßen Effektpigmente zeichnen sich durch eine Reihe von vorteilhaften Eigenschaften aus. Einerseits ist der Farbstoff in Form einer festhaftenden kompakten Schicht auf dem Interferenzpigment aufgebracht. Andererseits ist kein Haftvermittler, etwa ein Polymerbindemittelsystem zugeben. Beide Merkmale bewirken in Kombination eine dauerhafte Beschichtung des Farbstoffs auf dem Pigment, wobei die Körperfarbe des Farbstoffs in sehr hoher Farbechtheit und hoher Farbtiefe auch bei der Belegung in Form sehr dünner Schichten zu Geltung kommt. Darüber hinaus bleibt der eigentümliche Glanz des Interferenzpigments nach der Farbstoffbeschichtung nahezu vollständig erhalten. Bei den herkömmlichen Beschichtungen unter Zuhilfenahme von Haftvermittlern sind hingegen Glanzverluste und Abschwächung der Farbechtheit bzw. Farbtiefe zu beobachten. Die erfindungsgemäßen Effektpigmente zeichnen sich weiterhin durch einen Farb-Flop von Körperfarbe des Pigments zur Interferenzfarbe hin aus. Durch geeignete Kombination von Körperfarbe des Interferenzpigments mit der Körperfarbe des Farbstoffs können verschiedene Farbeffekte mit kräftigem Farbton erzielt werden, wobei letztlich ein hoher Glanz im fertiggestellten Pigment gewährleistet ist.

Zur Herstellung der erfindungsgemäßen Effektpigmente können alle geeigneten Interferenzpigmente, insbesondere die als Perlglanzpigmente bekannten, mit Metalloxiden beschichteten Glimmerschuppen verwendet werden. Prinzipiell sind alle üblichen Perlglanzpigmente geeignet, z.B. Glimmerbeschichtungen mit farblosen oder farbigen Metalloxiden wie $TiO_2$, $Fe_2O_3$, $SnO_2$, $Cr_2O_3$, ZnO und anderen Metalloxiden, allein oder in Mischung in einer einheitlichen Schicht oder in aufeinanderfolgenden Schichten. Diese Pigmente sind z.B. aus den deutschen Patenten und Patentanmeldungen 14 67 468, 19 59 998, 20 09 566, 22 14 545, 22 15 191, 22 44 298, 23 13 331, 25 22 572, 31 37 808, 31 37 809, 31 51 343, 31 51 354, 31 51 355, 32 11 602 und 32 35 017 bekannt.

Besonders bevorzugt werden mit farblosen Metalloxiden beschichtete Glimmerpigmente eingesetzt, die keine Körperfarbe sondern nur eine Interferenzfarbe besitzen. Zur Beschichung werden die Pigmente dann in der Farbstofflösung suspendiert. Die Wahl des geeigneten Lösungsmittels hängt dabei in der Regel vom Farbstoff selbst ab. Es können alle gängigen und dem Fachmann bekannten Phthalocyanin- und Metallphthalocyaninfarbstoffe wie sie beispielsweise in der EP 0 266 247 genannt sind, eingesetzt werden. Gewöhnlich werden 1 bis 20 Gew.%ige Farbstofflösungen verwendet. Ein besonders gut geeignetes Lösungsmittel ist konzentrierte Schwefelsäure.

In der Regel werden Beschichtungen mit etwa 1-3 % Farbstoff, bezogen auf das Gesamtpigmentgewicht, vorgenommen. Sollen spezielle Effekte erzielt werden, so kann der Farbstoffanteil auch außerhalb des genannten Bereichs liegen.

Die Suspension des Pigments in der Farbstofflösung wird anschließend mit einem Lösungsmittel, in welchem der Farbstoff im wesentlichen unlöslich ist, zusammengeführt. Im Falle einer Lösung des Farbstoffs in konzentrierter Schwefelsäure wird zweckmäßigerweise die Suspension durch Eingießen in Wasser überführt. Dabei wird der Farbstoff in Form einer festhaftenden kompakten Schicht auf das Pigment aufgefällt. Anschließend wird das Pigment nach herkömmlichen Verfahren abgetrennt, gewaschen und getrocknet. Ein nach dem erfindungsgemäßen Verfahren hergestelltes Effektpigment zeichnet sich durch hohen Glanz, verbesserte Farbstabilität und insbesondere durch den Farb-Flop von der Interferenzfarbe zur Körperfarbe des Pigments hin und durch verbesserte Dispergierbarkeit in Lacksystemen aus.

Die erfindungsgemäßen Effektpigmente können in beliebigen Formulierungen, in denen solche Pigmente zum Einsatz kommen, verwendet werden, beispielsweise in Lacken, Farbstoffen, Kunststoffen und Kosmetika.

Ausführungsbeispiele

Beispiel 1

1 g Nickelphthalocyanin (NiPc) werden in 100 ml konzentrierter Schwefelsäure unter Rühren gelöst. 9 g Iriodin® 100 der Fa. Merck, Darmstadt, werden in die Lösung eingetragen und unter Rühren suspendiert. Nach erfolgter Suspension und ca. 15 Minuten Nachrühren wird das Gemisch in etwa 1 l Eiswasser schnell und vollständig unter Rühren eingegossen. Das sich absetzende, mit Nickelphthalocyanin beschichtete Pigment wird abgesaugt, suflatfrei gewaschen und bei 80 °C im Trockenschrank getrocknet. Das so erhaltene Pigment wird in einer Konzentration von 7 %, bezogen auf das gesamte Lacksystem, in ein organishces Lackbindemittel (handelsüblicher Basislack der Fa. Herberts, Wuppertal) eingearbeitet. Als Ergebnis wird eine Lackierung erhalten, die einen blaugetönten Silbereffekt mit Farb-Flop zur blauen Körperfarbe des NiPc zeigt.

Beispiel 2

1 g Phthalocyanin (Pc) wird in 100 ml konzentrierter Schwefelsäure unter Rühren gelöst. 9 g des Interferenz-Pigments Iriodin® 235 der Fa. Merck, Darmstadt, werden in die Lösung eingetragen und suspendiert. Nach ca. 15 Minuten wird das Gemisch in etwa 1 l Eiswasser eingegossen. Das erhaltene, beschichtete Pigment wird abgesaugt, sulfatfrei gewaschen und bei 80 °C im Trockenschrank getrocknet. Das so hergestellte Perlglanzpigment wird in einer Konzentration von 7 %, bezogen auf das Gesamtlacksystem, in einem Klarlack (wie Beispiel 1) eingearbeitet. Als Ergebnis wird eine Lackierung erhalten, die einen grünen perlglänzenden Farbton als Interferenzfarbe des Iriodin® 235 mit einem Farb-Flop in die blaue Körperfarbe des Phthalocyanin zeigt.

**Patentansprüche**

1. Effektpigmente auf Basis von mit Phthalocyanin- oder Metallphthalocyaninfarbstoffen beschichteten Interferenzpigmenten, dadurch gekennzeichnet, daß die Farbstoffe ohne Zuhilfenahme von Bindemittelsystemen oder anderen Hilfsstoffen als fest anhaftende Schicht auf den Pigmenten aufgebracht sind.

2. Verfahren zur Herstellung von Efektpigmenten auf Basis von mit Phthalocyanin- oder Metallphthalocyaninfarbstoffen beschichteten Interferenzpigmenten, dadurch gekennzeichnet, daß man eine Suspension des Pigments in einer Lösung des Farbstoffs herstellt und die so erhaltene Suspension mit einem Lösungsmittel zusammenbringt, in welchem der Farbstoff zum überwiegenden Teil unlöslich ist.

3. Vewendung von Effektpigmenten gemäß Anspruch 1 in Formulierungen wie Lacken, Farbstoffen, Kunststoffen und Kosmetika.

4. Formulierungen enthaltend Effektpigmente gemäß Anspruch 1.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | EP-A-325484 ( KEMIRA OY) <br> * Seite 3, Zeilen 20 - 41 * <br> --- | 1 | C09C1/00 <br> C09D5/00 <br> A61K7/00 |
| X | EP-A-218156 (MERCK PATENT GMBH) <br> * Spalte 1, Zeilen 1 - 19; Ansprüche 1, 2 * <br> * Spalte 3, Zeilen 19 - 33 * <br> --- | 1 | C08K9/04 <br> C09B63/00 <br> C09C3/08 |
| X | EP-A-266247 ( RHONE-POULENC CHIMIE) <br> * Seite 5; Anspruch 1 * <br> --- | 1 | |
| A <br><br><br><br> D | CHEMICAL ABSTRACTS, vol. 105, no. 2, 14.07.1986 <br> Columbus, Ohio, USA <br> Seite 86; linke Spalte; ref. no. 8034W <br> * Zusammenfassung * <br> & CS-B-224792 (Nedorost, M. et al.) <br> --- | | |
| A | CHEMICAL ABSTRACTS, vol. 99, no. 12, 19 September 1983 <br> Columbus, Ohio, USA <br> Seite 81; linke Spalte; ref. no. 89678S <br> & JP-A-5821456 (Kubo, Yasushi) 08.02.1983 <br> * Zusammenfassung * <br> --- | | |
| A <br><br><br> D | World Patents Index (Latest) <br> Accession No. 84-104300, Week 17 <br> Derwent Publications Ltd, London GB <br> * Zusammenfassung * <br> & JP-A-59047240 (Mitsubishi Petroch. KK) <br> 16.03.1984 <br> ----- | | |

RECHERCHIERTE SACHGEBIETE (Int. Cl.5 )

C09C
C09D
A61K
C08K
C09B

Der vorliegende Recherchenbericht wurde für alle Patentansprüche erstellt

| Recherchenort | Abschlußdatum der Recherche | Prüfer |
|---|---|---|
| DEN HAAG | 20 JUNI 1991 | VAN BELLINGEN I. |